# FASCICULE DE BREVET EUROPEEN

(11) **EP 0 721 327 B1**
(45) Date de publication et mention de la délivrance du brevet: **09.06.1999**
(21) Numéro de dépôt: 94928438.4
(22) Date de dépôt: 27.09.1994
(51) Int. Cl.: A61K 9/12, A61K 9/70, A61L 25/00

(54) **COMPOSITION AEROSOL APTE A FORMER UNE MEMBRANE, DE PREFERENCE HYDRATEE, MEMBRANE, DE PREFERENCE HYDRATEE, AINSI OBTENUE ET SES APPLICATIONS, NOTAMMENT A TITRE DE PANSEMENT**
AEROSOL-ZUSAMMENSETZUNG GEEIGNET ZUR BILDUNG EINER VORZUGSWEISE HYDRIERTEN MEMBRAN, DERART ERHALTENE VORZUGSWEISE HYDRIERTE MEMBRAN UND IHRE ANWENDUNGEN, INSBESONDERE ALS VERBANDMATERIAL
AEROSOL COMPOSITION FOR FORMING A PREFERABLY HYDRATED MEMBRANE, PREFERABLY HYDRATED MEMBRANE SO OBTAINED, AND APPLICATIONS THEREOFPARTICULARLY AS A DRESSING

(30) Priorité: 28.09.1993 FR 9311761
(43) Date de publication de la demande: 17.07.1996
(73) Titulaire: FLAMEL TECHNOLOGIES, F-69693 Venissieux Cédex (FR)
(72) Inventeur: SOULA, Gérard, F-69330 Meyzieu (FR); GROSSELIN, Jean-Michel, F-69110 Ste.-Foy-lès-Lyon (FR); JORDA, Rafael, F-69110 Ste.-Foy-lès-Lyon (FR); CASTAN, Catherine, F-69530 Brignais (FR)
(74) Mandataire: Fleurance, Raphael
(86) Numéro de dépôt international: FR9401123
(87) Numéro de publication internationale: WO9508984

(56) Documents cités:
- EP-A- 0 327 411
- EP-A- 0 521 455
- EP-A- 0 560 014
- US-A- 4 920 158

## Description

Le domaine de la présente invention est celui des compositions aérosols et, plus particulièrement, de celles constituées par des filmogènes pulsés utiles, notamment mais non limitativement, pour l'application d'une pellicule protectrice curative sur des lésions, par exemple de la peau.

Plus précisément, la présente invention concerne une composition aérosol apte à former une membrane, de préférence hydratée.

L'invention a également pour objet la membrane, de préférence hydratée, obtenue à partir de la susdite composition aérosol.

Sans que cela ne soit limitatif, l'invention vise, plus spécifiquement, l'application de cette composition aérosol et de cette membrane, de préférence hydratée, pour le pansement de plaies, brûlures ou analogues.

### ART ANTERIEUR :

Le pansement de ces traumatismes localisés, souvent cutanés, a pour objet de les protéger vis-à-vis de l'environnement extérieur, de façon à éviter la contamination bactérienne et à permettre une cicatrisation rapide et de qualité. A cette fin, il convient que le pansement soit évidemment biocompatible, de manière à être parfaitement toléré et, de préférence, transparent pour permettre la surveillance aisée de l'état et de l'évolution du traumatisme.

Il est également avantageux que le pansement possède une certaine résistance mécanique, soit perméable à la vapeur d'au et soit, en outre, d'application et d'enlèvement aisé et indolore.

Le pansement le plus traditionnel est celui constitué de gaze (montée ou non sur bande plastique adhésive). L'inconvénient majeur de ce type de pansement est d'adhérer trop fortement à la plaie, de telle sorte que les changements de pansement, qui doivent être, somme toute, relativement fréquents, sont des opérations délicates et douloureuses. En outre, les lésions pansées sont, la plupart du temps, suppurantes, ce qui entraîne une occlusion de la gaze ou analogue. Il en résulte une imperméabilité du pansement, qui nuit à la cicatrisation et à la guérison de la lésion.

On connaît, par ailleurs, des filmogènes pulsés pour pansement, constitués par des compositions aérosols comprenant un polymère actif comme substance filmogène et apte à former, après vaporisation, une pellicule biocompatible, convenablement adhérente sur la peau, transparente et élastique, pour ne citer qu'une partie des exigences attendues dans l'application pansement et mentionnées ci-avant.

Il a déjà été proposé une grande variété de compositions aérosols contenant un polymère filmogène. On peut citer, par exemple :
- celle comportant du poly(acétate de vinyle), de la poly(vinylpyrrolidone) et de l'acide acétique (Cf. **FR- A-1 589 917**),
- celle à base de polysiloxane durcissable, du type bicomposant, associé à un gaz propulseur (Cf. **FR- A- 2 589 737**),
- celle constituée par des polyacrylates ou méthracrylates, de l'éthylcellulose, du polyvinylbutyral, un copolymère oxyde d'éthylène ou un polyisobutylène en solution dans un solvant, tel que le butanol (Cf. **FR-A-2 219 793** et **FR-A-2 212 134**),
- ou bien encore celle contenant un sel d'ammonium d'organosiliciés liés à des polymères organiques, tels que la poly(vinylpyrrolidone), l'acrylate ou le méthacrylate de cellulose, associé à un propulseur solvant (Cf. **US-A-4 921 691**).

Les polymères constitutifs de ces compositions aérosols connues présentent l'inconvénient de conduire à des films non hydratés et ne donnant pas entière satisfaction en ce qui concerne la cicatrisation. En outre, il est clair que ces polymères comprennent, selon toute vraisemblance, des monomères résiduels non naturels, susceptibles de générer des réactions inflammatoires, en raison de leur toxicité vis-à-vis d'un milieu vivant (e. g les dérivés acryliques et les siloxanes).

Pour tenter d'améliorer ces compositions aérosols vaporisables pour pansement, il a été proposé dans l'**EP-A-0 521 455** de substituer un polymère d'acide hydroxycarboxylique biodégradable aux polymères filmogènes imparfaits connus. Le polymère considéré peut être un polylactique et/ou un polyglycolique, dissous dans un solvant propulseur, tel que le diméthyléther, un fréon, un alcane gazeux, (e. g propane liquéfié) ou un analogue de ceux-ci. Cette composition aérosol comprend, en outre, de l'eau et un alcool, tel que l'éthanol, utile comme solvant de divers principes actifs thérapeutiques éventuellement présents.

Malgré les atouts que constituent leur biocompatibilité et leur biodégradabilité, ces polymères d'acides hydroxycarboxyliques souffrent d'un grave défaut lié à la qualité du film auquel ils sont susceptibles de conduire après vaporisation. Il s'avère, en effet, que les compositions aérosols à base de ces polymères d'acides hydroxycarboxyliques sont le siège d'une séparation de phase macroscopique lors de la vaporisation, ce qui a pour conséquence l'élimination de l'eau et l'obtention d'un film sec. Un tel produit apparaît comme n'étant pas extrêmement approprié pour le pansement de plaies, brûlures ou analogues, car son caractère trop anhydre entraîne, soit une difficulté d'adhésion à la plaie, soit une trop forte adhérence à celle-ci, ce qui provoque des difficultés et des douleurs lors du détachement du film.

On connaît, également, une autre demande de brevet européen (**EP-A-0 560 014**) qui décrit l'utilisation de polymères biodégradables, non réactifs, thermoplastiques et insolubles dans l'eau dissous dans un solvant miscible à l'eau. Après vaporisation de cette solution sur la zone à traiter, le procédé impose d'immerger cette zone dans l'eau, afin d'extraire le solvant et de former la membrane. Ce procédé conduit, certes, à une membrane aqueuse composée de polymère biodégradable, mais présente surtout les inconvénients majeurs suivants :
- la formation en deux étapes de la membrane fait perdre les avantages d'une forme spray qui sont, avant tout, formation instantanée, absence de manipulation, possibilité de traiter des zones difficiles en raison de leur relief ou de leur mauvaise accessibilité ...,
- les solvants miscibles dans l'eau cités dans le texte sont tous (mis à part l'acétone) des solvants lourds qui ne s'évaporent pas et ne sont pas extractibles de façon quantitative, à moins d'utiliser de grandes quantités d'eau ; ces solvants vont donc demeurer présents en partie dans le pansement au contact de la blessure. De plus, ces solvants sont en majorité non tolérés sur une lésion et génèrent, en général, une vive douleur.

Force est donc de constater qu'il existe un besoin en une composition aérosol à base de polymère biocompatible, biodégradable, non toxique et d'excipients pharmaceutiquement acceptables et permettant de produire par vaporisation, entre autres, des pansements se présentant sous la forme de films, ces derniers devant satisfaire à un cahier des charges propre à cette application :
- cohésion,
- perméabilité à la vapeur d'eau et à l'oxygène,
- imperméabilité aux bactéries,
- biocompatibilité, tolérance et non-toxicité,
- promoteur de cicatrisation,
- antalgique,
- adhérence convenable à la zone lésée, tout en gardant une aptitude au détachement aisé et sans douleur,
- de préférence, état hydraté apportant une certaine souplesse et une certaine élasticité et qui accélère le processus de guérison (M. F. JONKMAN in "High Performance Biomaterials", Ed. M. Szycher, Technomic (1991)).

On connaît une forme solide de pansement ou substitut cutané temporaire se présentant sous la fome d'une membrane souple, translucide, incolore et commercialisée sous la dénomination **INERPAN**® qui présente l'ensemble de ces qualités. Ce substitut cutané est constitué d'un copolymère de leucine et de glutamate de méthyle, imprégné d'un liquide à base de polyéthylène-glycol, de chlorure de sodium et d'eau purifiée.

Bien que l'I**NERPAN**® ait parfaitement démontré son efficacité pour le pansement, notamment de brûlures et d'escarres, il n'existe que sous forme de membranes épaisses de dimensions prédéterminées. En outre, la mise en place de ce pansement nécessite des précautions de manipulation et d'utilisation, qui limitent aujourd'hui son emploi au milieu hospitalier.

Il résulte de ce qui prédède, que la mise au point d'une formulation de polymère à base d'amino-acides, qui soit filmogène par vaporisation, serait un développement technique tout à fait souhaitable et intéressant, en particulier dans le domaine de la santé humaine et, notamment, en vue d'une application comme pansement.

La forme spray d'une membrane, de préférence hydratée, biocompatible constituerait un produit nouveau, tant dans la conception que dans la réalisation, et présenterait, par rapport aux pansements classiques non vaporisables à base de membranes hydrogels aujourd'hui connus, les avantages suivants :
- un coût unitaire de traitement peu élevé, en raison de la faible quantité de produit nécessaire par pansement, et une grande facilité d'utilisation, qui rendraient le produit adapté à une utilisation grand public,
- une variété et une flexibilité sur la dimension des pansements (surface et épaisseur).

En conséquence, l'un des objectifs essentiels de l'invention est de fournir une composition aérosol pharmaceutiquement acceptable, apte à former une membrane hydratée après vaporisation, ladite membrane pouvant être utilisée, notamment comme pansement, et possédant les propriétés évoquées ci-dessus et, en particulier, celles d'être un film cohésif, de préférence hydraté, biocompatible et thérapeutiquement performant. Pour atteindre cet objectif parmi d'autres, la Demanderesse a eu le mérite de résoudre, après de nombreux essais et études, le problème dont certains des aspects sont les suivants :
- rendre compatible un mélange d'une phase hydrophobe contenant un polymère filmogène et d'une phase hydrophile, de façon à éviter la précipitation du polymère et à permettre la vaporisation simultanée de ces deux phases pour l'obtention d'une membrane,
- doter cette membrane d'une bonne cohésion,
- et, de préférence, faire en sorte que cette membrane soit convenablement hydratée.

### EXPOSE SUCCINCT DE L'INVENTION :

La solution à ce problème consiste, notamment, à réaliser le mélange d'une phase hydrophobe contenant le polymère filmogène et d'une phase hydrophile et à sélectionner le polymère filmogène parmi les polyaminoacides.

### MEILLEURE MANIERE DE REALISER L'INVENTION :

La présente invention concerne ainsi une composition aérosol apte à former une membrane, de préférence hydratée, après vaporisation, caractérisée en ce qu'elle comprend :
- au moins une phase hydrophobe contenant un polyaminoacide hydrophobe, au moins en partie solubilisé dans un système solvant organique et choisi, de préférence, parmi les polyaminoacides obtenus à partir d'au moins l'un des acides aminés hydrophobes ou dérivés suivants : alanine, valine, leucine, isoleucine, proline, méthionine, phénylalanine, tryptophane, les esters des acides aspartique et glutamique,
- au moins une phase hydrophile, de préférence aqueuse,
- et au moins un propulseur.

Une telle composition aérosol est particulièrement avantageuse en tant que pansement, puisque, d'une part, son application sur la lésion s'effectue aisément et de manière indolore et, d'autre part, le film obtenu après pulvérisation contient au moins un produit hydrophile et/ou au moins un liquide, et/ou conserve, au moins pendant quelques minutes, l'eau présente initialement, de telle sorte qu'il se forme une membrane, de préférence hydratée.

Ce film de polymère, de préférence hydraté, est atoxique, non-irritant et éventuellement transparent. Il favorise la cicatrisation et se détache de la lésion aisément et sans douleur.

Ces résultats sont particulièrement surprenants et inattendus, dans la mesure où le (les) polyaminoacide(s) structurel(s), contenu(s) dans la phase hydrophobe, est (sont) incompatible(s) avec la phase aqueuse ou avec d'autres composants de la composition, tels que le propulseur. Il était donc difficile d'imaginer, a priori :
1 - comment vaporiser simultanément les deux phases hydrophobe et hydrophile,
2 - comment obtenir un film cohésif et non pas un amas de particules individuelles,
3 - comment le polymère pourrait piéger l'eau après vaporisation et évaporation du solvant.

Et pourtant, il s'avère qu'une simple agitation du mélange polyphasique, selon l'invention, permet d'obtenir une émulsion qui conduit, après vaporisation, à un film humide, cohésif et homogène. Contre toute attente, cette émulsion ne requiert pas l'incorporation de tensioactifs et, par ailleurs, on n'observe pas de précipitation du polymère. Il va de soi que l'invention n'en est pas pour autant limitée aux compositions sans tensioactifs. Ces derniers peuvent, en effet, être éventuellement des adjuvants desdites compositions.

Le polyaminoacide sélectionné conformément à l'invention peut être un homopolymère ou un copolymère d'acides aminés hydrophobes, de préférence, sélectionnés parmi ceux mentionnés ci-dessus. Ces homo et copolymères seront, indifféremment, désignés par le terme polymère dans le présent exposé.

Selon une modalité avantageuse de l'invention, la teneur en acides aminés hydrophobes dans le polyaminoacide est supérieure ou égale à 5 % en nombre et, de préférence, à 15 % en nombre.

Ces polymères ont un poids moléculaire suffisant pour conférer le corps et la texture de membrane aux matériaux obtenus par vaporisation de la composition aérosol de l'invention. Pour fixer les idées, on peut indiquer que ce poids moléculaire peut varier de 10³ à 15 x 10⁵ et, de préférence, de 4 x 10⁴ à 3 x 10⁵ D environ.

De façon privilégiée, on retient les polymères de leucine et/ou d'esters alkyliques, (de préférence, méthyle, éthyle ou benzyle) d'acide glutamique, en tant que polyaminoacide. Dans le cas où il s'agit d'un copolymère, par exemple Leu/Glu(OMe), les proportions relatives respectives, exprimées en moles, sont comprises entre 99/1 et 1/99, de préférence entre 30/70 et 70/30 et, plus préférentiellement encore, entre 45/55 et 55/45.

Conformément à l'invention, la composition aérosol peut comprendre un seul polymère ou un mélange de polymères.

S'agissant de la synthèse de ces polymères, il convient de préciser qu'elle est parfaitement connue en elle-même. Ainsi, les polymères mis en oeuvre dans la composition de l'invention peuvent être, par exemple, ceux décrits dans les demandes de brevet suivantes : **GB 996 760** et **FR 1 603 159**.

Le ou les polymères sont présents dans la composition aérosol à raison de 0,05 à 30 % poids/poids par rapport à la quantité totale d'aérosol.

Exprimée différemment, la concentration du polymère dans la phase organique hydrophobe est comprise entre 0,1 et 40, de préférence entre 0,5 et 10 et, plus préférentiellement encore, entre 0,5 et 4 % poids/volume par rapport au système solvant.

Suivant une modalité avantageuse de l'invention, le système solvant organique de la phase hydrophobe est constitué par au moins un éther, un halogénoalcane ou un halogénoalcène ou un halogénoaromatique ou bien encore un mélange d'entre eux.

En pratique, le choix du système solvant s'effectue, de préférence, parmi les composés suivants :
- les chlorofluorocarbures et analogues,
- les hydrogénofluorocarbures et analogues,
- les chlorocarbures et analogues,
- les acétals,
- les éthers,
- les esters,
- les cétones,
- les alcools,
- et les mélanges d'entre eux.
   le trichlorofluorométhane, le dichlorodifluorométhane, le chloro-1 di fluoro-1 éthane, le formiate de méthyle, le méthylal et le diméthyléther étant particulièrement préférés.

Selon une autre modalité avantageuse de l'invention, le rapport massique de la phase organique hydrophobe sur celui de la phase hydrophile, exprimé en partie en poids, est établi entre 100/1 et 1/1, de préférence entre 50/1 et 1/1 et, plus préférentiellement encore, entre 20/1 et 2/1.

Le propulseur utilisé pour assurer la vaporisation de la composition est, de préférence, constitué par au moins un gaz sous pression, liquéfié ou non, et sélectionné, de préférence, parmi la liste de produits suivants : propane, butane, isobutane, azote, CO₂, diméthyléther, halogénoalcanes (e. g chlorofluorocarbures ou analogues) et leurs mélanges.

Pour simplifier la composition, il est avantageux qu'au moins une partie du système solvant assume, à la fois, la fonction de solvant et la fonction de propulseur.

En pratique et dans le souci du respect de l'environnement, on peut utiliser des gaz et/ou des solvants non agressifs vis-à-vis de la couche d'ozone.

La quantité de propulseur n'est pas une donnée essentielle de la composition de l'invention mais on peut indiquer, à titre illustratif, qu'elle se situe généralement entre 1 et 99 % poids/poids, de préférence entre 40 et 90 % poids/poids par rapport à la quantité totale d'aérosol.

La phase hydrophile de la composition est, de préférence, aqueuse. Dans le cadre de l'optimisation de la composition aérosol de l'invention, il a été montré qu'il était particulièrement intéressant, dans certains cas, d'incorporer des adjuvants dans la phase aqueuse.

En particulier, la présence d'alcools inférieurs de **C**_{**1**} à **C**_{**10**}, tels que l'éthanol ou le propanol...., est parfois souhaitable pour améliorer la solubilisation de certains des constituants de la composition aérosol.

Parmi les susdits alcools inférieurs incorporables à la composition on sélectionne, de préférence, l'éthanol.

Certains adjuvants peuvent être utiles pour augmenter le pouvoir de rétention d'eau de la membrane, de préférence hydratée, obtenue après vaporisation de la composition aérosol de l'invention. Le choix de tels adjuvants fonctionnels peut se faire parmi les produits connus. Conformément à l'invention, on sélectionne plus préférablement les polyols, tels que le glycérol, les glycols ou les alcools "polymères", tels que les polyglycols, comme le polyéthylèneglycol ou le polypropylèneglycol, de masses moléculaires variables.

Les concentrations de ces adjuvants dans la composition sont avantageusement :
- de 0,1 à 10 %, de préférence de 0,5 à 5 %, pour ce qui concerne les alcools inférieurs,
- et de 0 à 50 %, de préférence 5 à 10 %, pour ce qui concerne les alcools polymères.

Pour parfaire l'efficacité thérapeutique de la composition aérosol de l'invention, dans le cadre de son application comme pansement, il est possible de lui adjoindre un ou plusieurs principes actifs de nature, par exemple, pharmaceutique ou cosmétique.

Cet ingrédient actif peut être, e.g, un désinfectant à usage externe, du type de ceux connus et/ou commercialisés, un agent bactéricide, fongicide ou virucide, un analgésique, un anti-inflammatoire, un hémostatique ou tout autre composé utile pour la préparation de médicaments à usage externe.

Il peut s'agir également de principes actifs cosmétiques, tels que des produits pour la protection solaire, voire même d'agents répulsifs des insectes.

Il est à noter que les adjuvants fonctionnels mentionnés précédemment peuvent servir pour la solubilisation de certains des susdits principes actifs.

Conformément à un mode préféré de réalisation de l'invention :
- la phase organique hydrophobe de la composition comprend :
   . un polymère de leucine et/ou d'un ester alkylique d'acide glutamique,
   . et un solvant constitué d'un mélange sous pression de diméthyléther et de méthylal, qui joue également le rôle de propulseur,
- tandis que sa phase hydrophile contient :
   . de l'eau,
   . et un polyol, de préférence un polyéthylèneglycol,
   ladite composition comportant, éventuellement, un ou plusieurs des principes actifs évoqués ci-dessus.

Il va de soi que cette composition peut être additionée de divers autres produits ou excipients traditionnellement employés dans les compositions aérosols.

Pour préparer cette composition aérosol, on procède de façon conventionnelle en mettant en contact le polymère et le solvant et en incorporant ensuite la phase aqueuse et le gaz propulseur sous pression. Le conteneur utilisé est une bombe aérosol classique, sertie juste avant injection du gaz propulseur liquide sous pression.

La vaporisation de cette composition, de préférence mise au préalable sous forme d'émulsion par agitation, s'opère de manière classique, par formation d'un brouillard de micro-gouttelettes de taille plus ou moins importante. Selon une variante et par extrapolation, la vaporisation peut être assimilée à une projection de liquide.

Suivant un autre de ses aspects, l'invention a pour objet la membrane, de préférence hydratée, obtenue par vaporisation de la composition aérosol décrite ci-avant. Il est clair que la composition aérosol est parfaitement adaptée et appropriée pour la mise sous forme de film hydraté, mais il est parfaitement envisageable de produire ce film à partir d'une mousse ou d'une crème.

Le film obtenu par vaporisation est, avantageusement, souple et humide. Cette humidité est, de préférence, au moins en partie obtenue par hydratation. La durée de l'hydratation dépend, d'une part, de la vitesse d'évaporation de l'eau, phénomène inévitable à température ambiante, et, d'autre part, du pouvoir rétenteur d'eau de la membrane, dont on a vu qu'il peut être modulé à l'aide d'adjuvants fonctionnels comme le polyéthylène glycol.

### APPLICATION INDUSTRIELLE :

Hormis l'application pansement (plaies, brûlures, dermatologie, traitement des coups de soleil, etc ...), la composition selon l'invention peut trouver des débouchés intéressants, notamment dans les domaines de la libération contrôlée de principes actifs (systèmes transdermiques) et du traitement de surface des biomatériaux (biocompatibilisation).

La présente invention sera mieux comprise, ses avantages et ses variantes de réalisation ressortiront bien des exemples qui suivent et qui décrivent la préparation de compositions aérosols et la production de pansements, de préférence hydratés, par vaporisation.

### EXAMPLES

### GÉNÉRALITÉS :

La composition aérosol, selon l'invention, est préparée dans les exemples qui suivent par mise en présence du polymère Leu/Glu(OMe) dans un gaz liquéfié sous pression. L'ajout de polyéthylène glycol (PEG) et d'eau (ou seulement l'un des deux) conduit à la formation d'une émulsion fluide que l'on peut facilement vaporiser. Cette émulsion décante en quelques heures mais on retrouve un mélange homogène en agitant le flacon. Il n'est pas nécessaire d'ajouter de tensioactifs pour obtenir cet effet. Lors de la vaporisation, une partie du système solvant s'évapore et la surface traitée est recouverte d'un film mince (environ 30 µm) et continu qui peut être plus ou moins humide et transparent selon la composition exacte de la formulation. Après environ 60 secondes, le film est suffisamment solide pour être soulevé et repositionné. Utilisé comme pansement, ce film adhère suffisamment à la peau et s'élimine aisément sous un jet d'au et, de surcroît, sans douleurs.

### EXEMPLE 1 :

**1.** On pèse 0,35 g de copolymère Leu/Glu(OMe) de composition molaire 49/51 et de viscosité réduite 0,8 dl/g.
**2.** On ajoute 27 ml de fréon 11 et 1,3 ml d'éthanol et on agite jusqu'à dissolution totale du polymère. On obtient une solution visqueuse et légèrement opaque.
**3.** On dilue la solution par ajout de 18 ml de fréon 11.
**4.** On ajoute 4,6 g d'eau et 7,6 g de PEG 600 à la solution de polymère, on sertit la bombe aérosol et on agite pour former l'émulsion.
**5.** On injecte sous pression 42 g de fréon 22. On agite.
Après pulvérisation, on obtient un film hydraté, souple et transparent.

### EXEMPLE 2 :

Mode opératoire selon l'exemple 1, où le copolymère est un copolymère Leu/Glu(OMe) 47/53 de viscosité réduite 1,9 dl/g.
On obtient un film hydraté, souple et transparent.

### EXEMPLE 3 :

Mode opératoire selon l'exemple 1, mais sans addition d'au.
On obtient une membrane souple et transparente.

### EXEMPLE 4 :

On pèse 1,01 g de polymère Leu/Glu(OMe) de composition molaire 47/53 et de viscosité réduite 2,0 dl/g. On ajoute 20,26 g de formiate de méthyle et 0,7 g de PEG 600. On sertit la valve de l'aérosol et on agite jusqu'à dissolution complète. On injecte alors 13,76 g de mélange standard butane/isobutane/propane (2,5 bars). On obtient, après pulvérisation sur la peau, un film continu, souple et résistant.

### EXEMPLE 5 :

On pèse 0,81 g de polymère Leu/Glu(OMe) de composition molaire 47/53 et de viscosité réduite 2,0 dl/g. On ajoute 14,94 g de méthylal (diméthoxyméthane) et on sertit la valve de l'aérosol. Après agitation jusqu'à dissolution complète, on injecte 30 g de diméthyléther, puis 4,20 g d'un mélange PEG 600/eau de composition 63/37 % p/p. On obtient, après pulvérisation sur la peau, un film continu, souple et hydraté.

### EXEMPLE 6 :

On pèse 1,01 g de polymère Leu/Glu(OMe) de composition molaire 47/53 et de viscosité réduite 2,0 dl/g. On ajoute 20,26 g de formiate de méthyle et 0,70 g de PEG 600. On sertit la valve de l'aérosol. Après agitation jusqu'à dissolution complète, on injecte sous pression 13,76 g d'un mélange butane-isobutane-propane 2,5 bars. On obtient, après pulvérisation sur la peau, un film continu et souple.

## Revendications

1. Composition aérosol apte à former une membrane, de préférence hydratée, après vaporisation, caractérisée en ce qu'eue comprend :
- au moins une phase hydrophobe contenant au moins un polyaminoacide hydrophobe, au moins en partie solubilisé dans un système solvant organique et choisi, de préférence, parmi les polyaminoacides obtenus à partir d'au moins l'un des acides aminés hydrophobes ou dérivés suivants : alanine, valine, leucine, isoleucine, proline, méthionine, phénylalanine, tryptophane, les esters des acides aspartique et glutamique,
- au moins une phase hydrophile, de préférence aqueuse,
- et au moins un propulseur.

2. Composition aérosol selon la revendication 1, caractérisée en ce que la teneur en acides aminés hydrophobes dans le polyaminoacide est supérieure ou égale à 5 % en nombre et, de préférence, à 15 % en nombre.

3. Composition aérosol selon la revendication 1 ou 2, caractérisée en ce que la concentration du polymère dans la phase organique hydrophobe est comprise entre 0,1 et 40, de préférence entre 0,5 et 10 et, plus préférentiellement, entre 0,5 et 4 % en poids/volume par rapport au système solvant.

4. Composition selon l'une quelconque des revendications 1 à 3 caractérisée en ce que le système solvant est constitué :
- par au moins un composé choisi parmi la classe des éthers et/ou des halogénoalcanes et/ou des halogénoalcènes et/ou des halogénoaromatiques,
- de préférence par au moins un chlorofluorocarbure ou analogue, un hydrogénofluorocarbure ou analogue, un chlorocarbure ou analogue, un acétal, un éther, un ester, une cétone, un alcool ou un mélange d'entre eux,
- le trichlorofluorométhane, ledichlorodifluorométhane, le chloro-1 difluoro-1-éthane, le formiate de méthyle, le méthylal et le diméthyléther étant particulièrement préférés.

5. Composition selon l'une quelconque des revendications 1 à 4, caractérisée en ce que le propulseur est sélectionné parmi les gaz sous pression, de préférence parmi la liste de produits suivants : propane, butane, isobutane, azote, CO₂, diméthyléther, halogénoalcanes et leurs mélanges.

6. Composition selon l'une quelconque des revendications 1 à 5, caractérisée en ce qu'au moins une partie du système solvant assume, à la fois, la fonction de solvant et la fonction de propulseur.

7. Composition selon l'une quelconque des revendications 1 à 6, caractérisée en ce que la phase aqueuse comporte des adjuvants, de préférence de nature alcoolique, et, plus préférentiellement encore, choisis parmi les produits suivants : éthanol, propanol, glycols, polyglycols, polyols ou leurs mélanges.

8. Composition selon l'une quelconque des revendications 1 à 7, caractérisée en ce que le rapport massique phase organique hydrophobe/phase hydrophile, exprimé en parties en poids, est établi entre 100/1 et 1/1, de préférence entre 50/1 et 1/1 et, plus préférentiellement encore, entre 20/1 et 2/1.

9. Composition selon l'une quelconque des revendications 1 à 8, caractérisée en ce qu'elle est additionnée d'au moins un principe actif, de préférence pharmaceutique ou cosmétique.

10. Composition selon l'une quelconque des revendications 1 à 9, caractérisée :
- en ce que sa phase organique hydrophobe comprend :
. un polymère de leucine et/ou d'un ester alkylique d'acide glutamique,
. et un solvant constitué d'un mélange sous pression de diméthyléther et de méthylal, qui joue également le rôle de propulseur,
- en ce que sa phase hydrophile contient :
. de l'eau,
. et un polyol, de préférence un polyéthylèneglycol,
- et en ce, qu'elle comporte, éventuellement, un ou plusieurs principes actifs.

11. Membrane, de préférence hydratée, caractérisée en ce qu'elle est obtenue par vaporisation de la composition aérosol selon l'une quelconque des revendications 1 à 10.

12. Pausement caractérisé en ce qu'il comprend la composition selon la revendication 11.

13. Système transdermique caractérisé en ce qu'il comprend une membrane selon la revendication 11.

## Claims

1. Aerosol composition for forming a preferably hydrated membrane after vaporization, characterized in that it comprises:
- at least one hydrophobic phase containing at least one hydrophobic polyamino acid which is at least partially solubilized in an organic solvent system and is preferably selected from polyamino acids obtained from at least one of the following hydrophobic amino acids or derivatives: alanine, valine, leucine, isoleucine, proline, methionine, phenylalanine, tryptophan and aspartic and glutamic acid esters,
- at least one, preferably aqueous, hydrophilic phase, and
- at least one propellant.

2. Aerosol composition according to Claim 1, characterized in that the content of hydrophobic amino acids in the polyamino acid is greater than or equal to 5% by number and preferably greater than or equal to 15% by number.

3. Aerosol composition according to Claim 1 or 2, characterized in that the concentration of the polymer in the hydrophobic organic phase is between 0.1 and 40, preferably between 0.5 and 10 and particularly preferably between 0.5 and 4% by weight/volume, based on the solvent system.

4. Composition according to any one of Claims 1 to 3, characterized in that the solvent system consists of:
- at least one compound selected from the class of ethers and/or halogenoalkanes and/or halogenoalkenes and/or halogenoaromatic compounds, and
- preferably at least one chlorofluorocarbon or analogue, one hydrogenofluorocarbon or analogue, one chlorocarbon or analogue, one acetal, one ether, one ester, one ketone, one alcohol or a mixture thereof,
trichlorofluoromethane, dichlorodifluoromethane, 1-chloro-1-difluoroethane, methyl formate, methylal and dimethyl ether being particularly preferred.

5. Composition according to any one of Claims 1 to 4, characterized in that the propellant is selected from pressurized gases and preferably from the following list of products: propane, butane, isobutane, nitrogen, CO₂, dimethyl ether, halogenoalkanes and mixtures thereof.

6. Composition according to any one of Claims 1 to 5, characterized in that at least part of the solvent system acts as both solvent and propellant simultaneously.

7. Composition according to any one of Claims 1 to 6, characterized in that the aqueous phase contains aduvants which are preferably of the alcohol type and are particularly preferably chosen from the following products: ethanol, propanol, glycols, polyglycols, polyols and mixtures thereof.

8. Composition according to any one of Claims 1 to 7, characterized in that the mass ratio of hydrophobic organic phase to hydrophilic phase, expressed in parts by weight, is set between 100/1 and 1/1, preferably between 50/1 and 1/1 and particularly preferably between 20/1 and 2/1.

9. Composition according to any one of Claims 1 to 8, characterized in that at least one, preferably pharmaceutical or cosmetic active principle is added thereto.

10. Composition according to any one of Claims 1 to 9, characterized in that:
- its hydrophobic organic phase comprises:
. a polymer of leucine and/or of a glutamic acid alkyl ester, and
. a solvent consisting of a pressurized mixture of dimethyl ether and methylal, which also acts as propellant,
- its hydrophilic phase contains:
. water, and
. a polyol, preferably a polyethylene glycol, and
- it optionally contains one or more active principles.

11. Preferably hydrated membrane, characterized in that it is obtained by vaporization of the aerosol composition according to any one of Claims 1 to 10.

12. Dressing, characterized in that it comprises the composition according to Claim 11.

13. Transdermal system, characterized in that it comprises a membrane according to Claim 11.

## Patentansprüche

1. Aerosol-Zusammensetzung, die in der Lage ist, nach Zerstäubung eine Membran, bevorzugt eine hydratisierte Membran, zu bilden, dadurch gekennzeichnet, daß sie umfaßt:
- mindestens eine hydrophobe Phase, die mindestens eine hydrophobe Polyaminosäure umfaßt, die zumindest teilweise in einem organischen Lösemittelsystem solubilisiert ist und vorzugsweise ausgewählt ist unter den Polyaminosäuren, die aus gehend von mindestens einer der folgenden hydrophoben oder derivatisierten Aminosäuren erhalten werden: Alanin, Valin, Leucin, Isoleucin, Prolin, Methionin, Phenylalanin, Tryptophan, den Estern von Asparaginsäure und Glutaminsäure,
- mindestens eine hydrophile, vorzugsweise wäßrige Phase,
- mindestens ein Treibmittel.

2. Aerosol-Zusammensetzung nach Anspruch 1, dadurch gekennzeichnet, daß der Gehalt an hydrophoben Aminosäuren in der Polyaminosäure über oder gleich 5% bezogen auf die Anzahl und vorzugsweise über oder gleich 15% bezogen auf die Anzahl beträgt.

3. Aerosol-Zusammensetzung nach Anspruch 1 oder 2, dadurch gekennzeichnet, daß die Konzentration des Polymers in der hydrophoben organischen Phase zwischen 0,1 und 40, vorzugsweise zwischen 0,5 und 10 und mehr bevorzugt zwischen 0,5 und 4 % (Gew./Vol.) bezogen auf das Lösemittelsystem beträgt.

4. Zusammensetzung nach einem der Ansprüche 1 bis 3, dadurch gekennzeichnet, daß das Lösemittelsystem gebildet wird:
- aus mindestens einer Verbindung, ausgewählt aus der Klasse der Ether und/oder der Halogenalkane und/oder der Halogenalkene und/oder der halogenierten Aromaten,
- vorzugsweise aus mindestens einem Chlorfluorkohlenwasserstoff oder Analog davon, einem Wasserstofffluorkohlenwasserstoff oder Analog davon, einem Chlorkohlenwasserstoff oder Analog davon, einem Acetal, einem Ether, einem Ester, einem Keton, einem Alkohol oder einer Mischung von diesen,
- wobei Trichlorfluormethan, Dichlordifluormethan, Chlor-1-difluor-1-ethan, Methylformiat, Methylal und Dimethylether besonders bevorzugt sind.

5. Zusammensetzung nach einem der Ansprüche 1 bis 4, dadurch gekennzeichnet, daß das Treibmittel ausgewählt wird aus den unter Druck stehenden Gasen, vorzugsweise aus der folgenden Liste von Produkten: Propan, Butan, Isobutan, Stickstoff, CO₂, Dimethylether, Halogenalkanen und deren Mischungen.

6. Zusammensetzung nach einem der Ansprüche 1 bis 5, dadurch gekennzeichnet, daß mindestens ein Teil des Lösemittelsystems zugleich die Lösemittel-Funktion und die Treibmittel-Funktion übernimmt.

7. Zusammensetzung nach einem der Ansprüche 1 bis 6, dadurch gekennzeichnet, daß die wäßrige Phase Zusatzstoffe umfaßt, die vorzugsweise von alkoholischer Natur sind und noch mehr bevorzugt ausgewählt werden unter den folgenden Produkten: Ethanol, Propanol, Glykolen, Polyglykolen, Polyolen oder deren Mischungen.

8. Zusammensetzung nach einem der Ansprüche 1 bis 7, dadurch gekennzeichnet, daß das Massenverhältnis hydrophobe organische Phase/hydrophile Phase, ausgedrückt in Gewichtsteilen, zwischen 100/1 und 1/1, vorzugsweise zwischen 50/1 und 1/1 und noch mehr bevorzugt zwischen 20/1 und 2/1 eingestellt wird.

9. Zusammensetzung nach einem der Ansprüche 1 bis 8, dadurch gekennzeichnet, daß ihr mindestens ein Wirkstoff, vorzugsweise ein pharmazeutischer oder kosmetischer, zugesetzt wird.

10. Zusammensetzung nach einem der Ansprüche 1 bis 9, dadurch gekennzeichnet:
- daß deren hydrophobe organische Phase umfaßt:
• ein Polymer von Leucin und/oder einem Alkylester von Glutaminsäure,
• und ein Lösemittel, das aus einer unter Druck stehenden Mischung von Dimethylether und Methylal gebildet wird, das gleichfalls die Rolle eines Treibmittels spielt,
- daß deren hydrophile Phase enthält:
• Wasser,
• ein Polyol, vorzugsweise ein Polyethylenglykol,
- und daß sie gegebenenfalls einen oder mehrere Wirkstoffe umfaßt.

11. Membran, die bevorzugt hydratisiert ist, dadurch gekennzeichnet, daß sie durch Zerstäuben der Aerosol-Zusammensetzung nach einem der Ansprüche 1 bis 10 erhalten wird.

12. Verband, dadurch gekennzeichnet, daß er die Zusammensetzung nach Anspruch 11 umfaßt.

13. Transdermalsystem, dadurch gekennzeichnet, daß es eine Membran nach Anspruch 11 umfaßt.
